# DEMANDE DE BREVET EUROPEEN

(11) **EP 4 796 158 A1**
(43) Date de publication de la demande: **26.08.2026**
(21) Numéro de dépôt: 26153904.3
(22) Date de dépôt: 23.01.2026
(51) Int. Cl.: A61M 39/10, A61M 39/12, A61M 5/14

(54) **COUPLAGE D'UN CATHÉTER À UNE POMPE DE PERFUSION DE MÉDICAMENT LIQUIDE**

(30) Priorité: 19.02.2025 FR 2501702
(71) Demandeur: L'Air Liquide, Société Anonyme pour l'Etude et l'Exploitation des Procédés Georges Claude, 75007 Paris (FR)
(72) Inventeur: SCHMITT, Mary, 75007 Paris (FR)
(74) Mandataire: Air Liquide

(57) **Abrégé**

L'invention concerne un ensemble (1) d'administration d'un médicament liquide, de préférence de l'insuline, comprenant une pompe (10) de perfusion comprenant un réservoir (11) pour stocker le médicament liquide, et un premier connecteur (13) comprenant un taraudage (14), en communication fluidique avec le réservoir (11), et un cathéter (20) comprenant un tuyau flexible (21) équipé d'un second connecteur (23) comprenant un filetage (24) complémentaire du taraudage (14) du premier connecteur (13) de manière à pouvoir coupler par vissage les premier et second connecteurs (13, 23), l'un à l'autre, et ainsi raccorder fluidiquement le cathéter (20) à la pompe (10). Le couplage par vissage des premier et second connecteurs (13, 23), l'un à l'autre, comprend un déplacement en rotation (R) relatif de l'un par rapport à l'autre, selon un angle de rotation compris entre 100° et 200°.

## Description

L'invention concerne un ensemble d'administration d'un médicament liquide, telle de l'insuline, comprenant une pompe de perfusion et un cathéter servant à acheminer le médicament liquide.

Les pompes de perfusion sont des dispositifs médicaux d'administration de fluides utilisées pour administrer un ou plusieurs fluides, typiquement un médicament sous forme liquide, i.e. un médicament liquide, aux personnes en ayant besoin dans le cadre d'un traitement médical, c'est-à-dire à des patients. Le médicament liquide est généralement stocké dans un réservoir agencé dans la pompe de perfusion.

Ainsi, des pompes de perfusion sont couramment mises en œuvre afin d'administrer de l'insuline aux patients diabétiques afin de réguler le taux de glucose dans le sang. Selon le modèle, les pompes de perfusion peuvent être portées par le patient en étant fixées à la ceinture, placées dans un sac, une pochette, un brassard ou analogue, ou encore être collées directement sur la peau du patient.

De telles pompes de perfusion sont décrites par EP2155288, EP4491206, EP1605993, US2002/0169439 et WO2005/039673.

Afin de permettre l'administration du médicament liquide au patient, typiquement en sous-cutané ou autre, certaines pompes de perfusion doivent être raccordées à un système de perfusion comprenant un cathéter alimenté par le réservoir de la pompe servant à acheminer le médicament liquide jusqu'à un hub ou une « plateforme » de perfusion comprenant une canule d'injection ou analogue, et de préférence des moyens adhésifs permettant de maintenir le hub de perfusion collé sur la peau du patient. De tels systèmes de perfusion sont décrits par EP3352814, WO98/58693 et US2006/0264890.

Le système de perfusion, en particulier le cathéter, est un élément consommable qui doit être régulièrement remplacé pour des raisons évidentes d'hygiène et de sécurité sanitaire pour le patient.

Or, le raccordement du cathéter à la pompe de perfusion est un problème récurrent pour les patients, en particulier chez certains patients présentant des complications liées à leur diabète, par exemple des complications visuelles ou micro ou macro vasculaires réduisant leur dextérité, pouvant engendrer de mauvaises connexions engendrant des fuites inévitables de médicament liquide, ce qui peut affecter négativement leur traitement thérapeutique.

EP1958660 a proposé un raccordement via des connecteurs mâle et femelle se fixant l'un à l'autre via un système à baïonnette incluant un élément élastique, c'est-à-dire un ressort ou analogue, agencé entre les deux connecteurs. Un tel système n'est pas idéal car de conception assez complexe et le système à baïonnette peut poser des difficultés à certains patients, en particulier les personnes âgées ou présentant un handicap (e.g. visuel...).

On connait par ailleurs :
- US5,545,152 enseignant un dispositif de raccordement rapide pour système d'infusion de médicament comprenant des connecteurs mâle et femelle se couplant l'un à l'autre. Le connecteur mâle porte sur sa périphérie externe des « plots » en saillie et le connecteur femelle comporte des « fenêtres » traversant sa paroi périphérique. Une fois insérés l'un dans l'autre, une rotation relative des connecteurs selon un angle de 90° permet d'insérer les plots en saillie dans les fenêtres afin d'assurer le verrouillage des connecteurs. Il est nécessaire de prévoir des surfaces formant cames sur les parois internes du connecteur femelle afin d'assurer ladite insertion. En cas de rotation insuffisante et/ou de mauvaise insertion des plots dans les fenêtres associées, les connecteurs mâle et femelle peuvent se découpler intempestivement. De plus, du fait de l'effet des cames, les plots en saillie peuvent se dégrader ou s'éroder au fil du temps, i.e. des couplages/découplages, conduisant à des verrouillages insuffisants.
- US9,227,047 qui propose un dispositif de raccordement rapide comprenant plusieurs éléments raccordés les uns aux autre. Du côté proximal, des éléments mâle et femelle se couplent l'un à l'autre par vissage selon un système de type Luer-Lock. Une pièce interne déformable percée assure l'étanchéité fluidique et par ailleurs un acheminement du fluide à l'intérieur du dispositif. En outre, du côté distal, un élément-pousseur mobile coopère avec des pièces-clapets internes pour permettre ou interdire la sortie de fluide. L'architecture globale de ce dispositif est complexe et peu adaptée aux personnes présentant des complications liées à leur diabète.
- US2016/0354288 qui décrit un dispositif de raccordement rudimentaire de type Luer-Lock avec connecteurs mâle et femelle se raccordant l'un à l'autre par vissage adapté à la nutrition entérale des personnes ne pouvant s'alimenter suffisamment par voie orale. Il n'est pas conçu pour l'administration d'insuline à des personnes diabétiques.
- US2022/0062616 qui enseigne un dispositif de raccordement rudimentaire comprenant des connecteurs mâle et femelle se raccordant l'un à l'autre par vissage. Le pas de vis doit être relativement long pour assurer un bon couplage des connecteurs. Le vissage se fait sur 1 tour ou plusieurs tours (i.e. au moins 360°). Il n'est pas bien adapté aux personnes présentant des complications liées à leur diabète.

Un problème est donc de faciliter le raccordement d'un cathéter à une pompe de perfusion de manière à assurer un raccordement efficace et une bonne étanchéité fluidique, y compris chez les patients susmentionnés présentant des complications liées à leur diabète.

Une solution selon l'invention concerne alors un ensemble d'administration d'un médicament liquide, de préférence d'insuline, comprenant :
- une pompe de perfusion comprenant un réservoir pour stocker le médicament liquide, et un premier connecteur, aussi appelé « connecteur femelle », comprenant un taraudage, en communication fluidique avec le réservoir, i.e. de manière à pouvoir prélever et/ou délivrer au moins une partie du médicament liquide stocké dans le réservoir, et
- un cathéter comprenant un tuyau flexible équipé d'un second connecteur, aussi appelé « connecteur mâle », comprenant un filetage complémentaire du taraudage du premier connecteur (i.e. le connecteur femelle) de la pompe de manière à pouvoir coupler par vissage les premier et second connecteurs (i.e. des connecteurs mâle et femelle) l'un à l'autre et ainsi raccorder fluidiquement du cathéter à la pompe de perfusion, en particulier au réservoir de la pompe.

De plus, le premier connecteur équipant la pompe comprend un corps tubulaire agencé coaxialement (XX) autour d'une tubulure interne en étant espacé l'un de l'autre en définissant une chambre interne, ladite tubulure interne comprenant un passage interne débouchant au niveau d'une sortie de fluide faisant face à l'extrémité avant ouverte de la chambre interne du corps tubulaire.

Par ailleurs, le second connecteur équipant le cathéter comprend une portion avant tubulaire et un passage de fluide interne reliant fluidiquement une entrée de fluide agencée sur la portion avant tubulaire, au tuyau flexible du cathéter.

En outre, dans l'ensemble d'administration de médicament liquide selon l'invention, le taraudage est agencé dans la paroi interne du corps tubulaire du premier connecteur équipant la pompe, alors que le filetage est porté par la surface périphérique externe de la portion avant tubulaire du second connecteur équipant le cathéter.

Dès lors, lorsque les premier et second connecteurs sont couplés l'un à l'autre :
- le filetage du second connecteur et le taraudage du premier connecteur coopèrent l'un avec l'autre, pour assurer le couplage par vissage des premier et second connecteurs, l'un à l'autre, avec déplacement en rotation (R) relatif de l'un par rapport à l'autre, selon un angle de rotation compris entre 100° et 200°,
- et la tubulure interne du premier connecteur vient se loger de manière étanche, dans la portion avant tubulaire du second connecteur.

Autrement dit, selon l'invention, le couplage par vissage des premier et second connecteurs (i.e. des connecteurs mâle et femelle), l'un à l'autre, comprend un déplacement en rotation relatif de l'un par rapport à l'autre (i.e. course angulaire), selon un angle de rotation compris entre environ 100° et 200°, de préférence entre 100° et 190° environ.

Selon le mode de réalisation considéré, l'ensemble d'administration de médicament liquide selon l'invention peut comprendre l'une ou plusieurs des caractéristiques suivantes :
- l'angle de rotation est inférieur ou égal à 190°.
- l'angle de rotation est inférieur ou égal à 185°, de préférence inférieur ou égal à 180°.
- l'angle de rotation est inférieur à 170°, de préférence inférieur à 160°.
- l'angle de rotation est d'au moins 105°.
- l'angle de rotation est d'au moins 110°, de préférence d'au moins 120°.
- l'angle de rotation est d'au moins 125°, de préférence d'au moins 130°.
- l'angle de rotation est d'au moins 135°, de préférence d'au moins 140°.
- avantageusement, l'angle de rotation est compris entre 130° et 195°, de préférence inférieur ou égal à 190°.
- l'angle de rotation est préférentiellement compris entre 130° et 160°, de préférence encore compris entre 140° et 155°.
- le premier connecteur de la pompe de perfusion est agencé à une extrémité libre d'un conduit flexible relié fluidiquement au réservoir.
- le conduit flexible de la pompe est raccordé fluidiquement à une sortie du réservoir contenant le médicament liquide.
- la pompe comprend un système (i.e. mécanisme) de dosage agencé entre la sortie du réservoir de la pompe et l'entrée du premier connecteur ou l'entrée du conduit flexible portant le premier connecteur, en particulier de micro-dosage.
- au moins une partie du système (i.e. mécanisme) de dosage est agencé sur le réservoir de la pompe.
- le médicament liquide contenu dans le réservoir est de préférence de l'insuline ou un autre médicament sous forme liquide.
- selon un mode de réalisation, le passage de fluide interne du second connecteur comprenant une portion tronconique interne.
- le passage de fluide interne du second connecteur est cylindrique avec une portion terminale tronconique interne .
- selon un autre mode de réalisation, le passage de fluide interne du second connecteur est cylindrique ou approximativement cylindrique, typiquement entièrement cylindrique.
- le premier connecteur équipant la pompe comprend un premier axe (XX).
- le premier connecteur a une forme générale allongée selon le premier axe (XX), de préférence une forme tubulaire (i.e. un corps tubulaire allongé).
- le second connecteur équipant le cathéter comprend un second axe (YY).
- le second connecteur a une forme générale tubulaire allongée selon le second axe (YY).
- le premier connecteur équipant la pompe comprend un corps tubulaire comprenant une paroi externe texturée, par exemple striée ou autre, pour faciliter sa tenue par l'utilisateur, typiquement entre ses doigts.
- le premier connecteur équipant la pompe comprend un corps tubulaire de forme générale cylindrique, i.e. de révolution.
- le premier connecteur équipant la pompe comprend une portion arrière tubulaire (i.e. une première portion arrière), à laquelle est raccordée fluidiquement à l'extrémité libre du conduit flexible.
- la portion arrière tubulaire est solidaire du corps tubulaire du premier connecteur, en particulier d'une paroi de fond du corps tubulaire, de préférence formés d'une pièce unique, par exemple obtenue par injection-moulage ou analogue.
- le diamètre externe de la portion arrière tubulaire est inférieur à celui du corps tubulaire externe du premier connecteur.
- le corps tubulaire du premier connecteur délimite un volume ou une chambre interne.
- la tubulure interne du premier connecteur est agencée dans le volume ou chambre interne.
- la tubulure interne du premier connecteur équipant la pompe comprend un passage interne débouchant au niveau d'une sortie de fluide, de préférence débouchant dans le volume interne.
- la tubulure interne du premier connecteur comprend une portion terminale comprenant la sortie de fluide.
- selon un mode de réalisation, la portion terminale est de forme tronconique.
- au moins une partie de la portion terminale de forme tronconique du premier connecteur est complémentaire d'au moins une partie de la portion tronconique interne du passage de fluide interne du second connecteur de sorte que ceux-ci puissent se coupler de manière étanche l'un à l'autre, i.e. avec coopération étanche des portions tronconiques l'une avec l'autre, après couplage.
- selon un mode de réalisation, la rotation relative des connecteurs l'un par rapport à l'autre est stoppée lorsque les portions complémentaires, notamment tronconiques, sont insérées de manière étanche l'une dans l'autre.
- selon un mode de réalisation, la portion terminale est de forme cylindrique ou approximativement cylindrique.
- au moins une partie de la portion terminale du premier connecteur est complémentaire d'au moins une partie du passage de fluide interne du second connecteur de sorte que ceux-ci puissent se coupler de manière étanche l'un à l'autre, i.e. avec coopération étanche des portions complémentaires l'une avec l'autre, après couplage.
- la portion arrière tubulaire du corps tubulaire du premier connecteur est traversé par un canal interne en communication fluidique avec le passage interne de la tubulure interne, de préférence via un orifice.
- le corps tubulaire du premier connecteur comprend une extrémité avant ouverte comprenant une ouverture avant, i.e. une ouverture large.
- le corps tubulaire du premier connecteur comprend une extrémité arrière, aussi appelée un fond, fermée par une paroi de fond.
- le corps tubulaire du premier connecteur a une forme générale de coupelle ou analogue, c'est-à-dire présentant une paroi périphérique se raccordant à un fond, notamment un fond plat en forme de disque.
- lorsque les premier et second connecteurs sont couplés l'un à l'autre, le filetage du second connecteur et le taraudage du premier connecteur coopèrent l'un avec l'autre de manière à assurer leur vissage.
- lorsque les premier et second connecteurs sont couplés l'un à l'autre, la tubulure interne du premier connecteur vient se loger de manière étanche, dans la portion avant tubulaire du second connecteur.
- de préférence, la tubulure interne du premier connecteur vient se loger de manière étanche dans le passage de fluide interne du second connecteur, via l'entrée de fluide de la portion avant tubulaire du second connecteur.
- lorsque les premier et second connecteurs sont couplés l'un à l'autre, ils sont coaxiaux (XX, YY).
- le second connecteur comprend en outre une portion arrière (i.e. une seconde portion arrière), conformée pour être saisie à 2 doigts par l'utilisateur, en particulier être tenue entre pouce et index.
- la portion arrière du second connecteur a une forme aplatie.
- la portion arrière du second connecteur comprend des zones aplaties à surface plane ou courbée, par exemple deux zones aplaties situées de part et d'autre de la portion arrière.
- la portion arrière du second connecteur est solidaire de la portion avant tubulaire.
- le second connecteur comprend un épaulement annulaire, en particulier porté par la portion arrière tubulaire.
- l'épaulement annulaire du second connecteur vient se positionner en regard d'une bordure annulaire du premier connecteur, lorsque les premier et second connecteurs sont couplés l'un à l'autre, i.e. l'une face à l'autre.
- l'extrémité avant ouverte du premier connecteur est délimitée par la bordure annulaire.
- la rotation relative des connecteurs l'un relativement à l'autre se fait dans le sens horaire pour opérer leur couplage, i.e. vissage, et dans le sens antihoraire pour opérer leur découplage, i.e. dévissage, ou inversement.
- le premier connecteur et/ou le second connecteur comprennent des moyens d'arrêt de rotation configurés pour stopper la rotation (i.e. vissage) relative des connecteurs l'un relativement à l'autre, lors de leur couplage.
- les moyens d'arrêt de rotation comprennent une (ou plusieurs) butée(s), épaulement(s) ou autres.
- selon un mode de réalisation, les moyens d'arrêt de rotation comprennent une conformation ou configuration spécifique du filetage et/ou du taraudage des connecteurs.
- selon un mode de réalisation, la conformation ou configuration spécifique du filetage et/ou du taraudage des connecteurs assure également leur verrouillage ou blocage en position l'un dans l'autre.
- l'épaulement annulaire et la bordure annulaire des connecteurs viennent se positionner en regard l'un de l'autre, c'est-à-dire face à face.
- selon un mode de réalisation, l'épaulement annulaire et la bordure annulaire des connecteurs viennent se positionner en butée ou quasi-butée, l'un contre l'autre.
- selon un mode de réalisation, lorsque l'épaulement annulaire et la bordure annulaire viennent en butée l'un contre l'autre, la rotation relative des connecteurs l'un relativement à l'autre est stoppée.
- le passage de fluide interne du second connecteur équipant le cathéter relie fluidiquement l'entrée de fluide agencée sur la portion avant tubulaire à une sortie de fluide agencée dans la portion arrière du second connecteur.
- lorsque les premier et second connecteurs sont couplés l'un à l'autre, le volume ou chambre interne du premier connecteur contient de l'air, c'est-à-dire qu'elle ne contient pas ou jamais de médicament liquide, par exemple d'insuline.
- la passage de fluide interne traverse le second connecteur selon le second axe (YY).
- le cathéter comprend un tuyau souple en polymère.
- le conduit flexible est en polymère.
- le réservoir a une contenance en fluide comprise entre 1 et 5 ml, typiquement entre 1,5 et 3,5 ml.
- de préférence les premier et second connecteurs sont transparents, i.e. en polymère transparent.
- le premier et/ou le second connecteurs sont formés d'un polymère de type butadiène styrène, exemple le méthyl métacrylate acrylonitrile butadiène styrène (MABS).
- la pompe de perfusion comprend un système de dosage agencé en sortie de réservoir.
- le système de dosage de la pompe est associé au réservoir de manière à pouvoir extraire une ou des doses de médicament liquide dudit réservoir.
- le système de dosage de la pompe est motorisé, i.e. moteur électrique, et/ou commandé par une électronique de commande.
- le réservoir de la pompe est amovible, c'est-à-dire extractible.
- le réservoir de la pompe comprend un boitier ou enveloppe externe rigide, par exemple en polymère.
- le boitier rigide du réservoir contient une enveloppe ou poche interne souple, par exemple formé d'une (ou plusieurs) feuille en polymère.
- la poche interne souple est dimensionnée pour que sa paroi externe vienne au contact ou quasi-contact de la paroi interne du boitier rigide du réservoir, lorsque le réservoir est rempli de médicament liquide, typiquement d'insuline.
- le boitier rigide du réservoir est fermé par un couvercle ou analogue comprenant au moins un orifice de sortie, de préférence le couvercle porte un conduit comprenant l'orifice de sortie.
- la poche interne souple est destinée à recevoir, i.e. stocker, le médicament liquide, e.g. de l'insuline.

L'invention concerne en outre le cathéter d'un ensemble d'administration de médicament liquide, de préférence de l'insuline, selon l'invention comprenant un tuyau flexible équipé, d'une part, du second connecteur et, d'autre part, d'un système de couplage à une plateforme de perfusion/infusion comprenant une aiguille de perfusion destinée à être insérée dans les tissus d'un patient afin d'y délivrer le médicament liquide.

L'invention concerne aussi la pompe de perfusion d'un ensemble d'administration d'un médicament liquide, de préférence de l'insuline, selon l'invention, comprenant le premier connecteur.

Dès lors, l'invention concerne aussi l'ensemble ou kit de perfusion de médicament liquide comprenant la pompe de perfusion et le cathéter d'un ensemble d'administration de médicament liquide selon l'invention, et une plateforme (ou hub) de perfusion comprenant une aiguille (ou canule) de perfusion, i.e. d'administration ou injection, servant à délivrer le médicament liquide, dans lequel ladite aiguille de perfusion est en communication fluidique avec le réservoir de la pompe de perfusion, via le cathéter, de manière à acheminer le médicament liquide, typiquement de l'insuline, du réservoir à l'aiguille servant à l'administration du médicament liquide.

Enfin, l'invention concerne aussi un procédé de couplage d'un cathéter à une pompe de perfusion d'un ensemble d'administration de médicament liquide, de préférence de l'insuline, selon l'invention, dans lequel un utilisateur :
a. opère d'abord un rapprochement, de préférence en translation, des premier et second connecteurs (i.e. des connecteurs mâle et femelle) l'un de l'autre jusqu'à insérer (de préférence partiellement) l'un des connecteurs dans l'autre, et
b. réalise ensuite un couplage par vissage des premier et second connecteurs, l'un à l'autre, (i.e. coopération du filetage et du taraudage) avec déplacement en rotation relatif de l'un par rapport à l'autre (i.e. course angulaire), selon un angle de rotation compris entre environ 100° et 200°, de préférence entre 110° et 190° environ.

De préférence, le vissage de l'étape ii) se fait en maintenant le premier connecteur et/ou en tournant le second connecteur dans le sens horaire.

De préférence, on stoppe le vissage :
- lorsque les portions complémentaires, par exemple tronconiques, de la tubulure interne du premier connecteur et du passage de fluide interne du second connecteur sont associées l'une à l'autre, i.e. insérées de manière étanche l'une dans l'autre, et/ou
- lorsque le filetage et le taraudage des connecteurs sont vissés au maximum l'un dans l'autre, c'est-à-dire lorsqu'aucun vissage supplémentaire ne peut être opéré par l'utilisateur (e.g. existence d'une résistance au vissage supplémentaire).

L'invention va maintenant être mieux comprise grâce à la description détaillée suivante, faite à titre illustratif mais non limitatif, en référence aux figures annexées parmi lesquelles :
- Figure 1 schématise un mode de réalisation d'un ensemble d'administration d'un médicament liquide, telle de l'insuline, comprenant un cathéter raccordé à une pompe de perfusion selon la présente invention.
- Figure 2 schématise (vue en coupe) un mode de réalisation d'un second connecteur équipant le cathéter de Fig. 1 selon la présente invention.
- Figure 3 schématise (vue en coupe) un mode de réalisation d'un premier connecteur équipant la pompe de perfusion de Fig. 1 selon la présente invention.
- Figure 4 représente une vue de face schématique du premier connecteur de Fig. 3.
- Figure 5 schématise (vue en coupe) le couplage avec raccordement fluidique des premier et second connecteurs selon la présente invention, schématisés en Fig. 2 et Fig. 3.

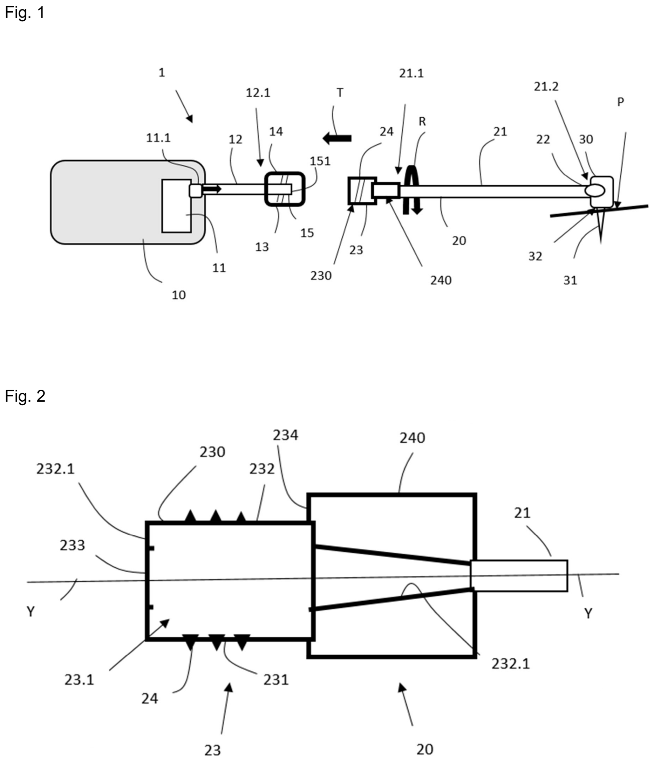

Fig. 1 schématise un mode de réalisation d'un ensemble d'administration d'un médicament liquide 1, à savoir ici de l'insuline, selon la présente invention comprenant une pompe 10 de perfusion équipée d'un réservoir 11 servant à recevoir et conserver le médicament liquide en vue de sa distribution à une personne en ayant besoin dans le cadre d'un traitement, c'est-à-dire un patient, par exemple une personne diabétique ayant besoin d'injections d'insuline (pluri-)quotidiennes.

Dans le reste de la description et à des fins de simplification, le médicament liquide est considéré comme étant de l'insuline. Toutefois, dans le cadre de l'invention, d'autres médicaments liquides peuvent être contenus dans le réservoir 11 et fournis au patient par la pompe de perfusion 10 dans le cadre d'autres traitements thérapeutiques.

Le réservoir 11 de la pompe de perfusion 10 est préférentiellement extractible, c'est-à-dire qu'il peut être retiré de la pompe 11, lorsqu'il est vide et/ou usagé, pour être (re-)rempli avec du médicament liquide i.e. ici de l'insuline, ou pour être remplacé par un autre réservoir plein, i.e. un réservoir neuf. Le réservoir 11 rempli/plein peut ensuite être réinséré dans un logement à réservoir aménagé dans la pompe 10. En général, un tel réservoir 11 a une contenance de 1 à 5 ml ou plus, typiquement de 1 à 3,5 ml environ.

Le réservoir 11 peut être au moins partiellement souple ou rigide, ou les deux. Par exemple, le réservoir de la pompe peut comprendre un boitier externe rigide, par exemple en polymère, contenant une poche interne souple, i.e. flexible, par exemple formé d'une ou plusieurs feuilles en polymère. Avantageusement, la poche interne peut avoir une paroi transparente. Le médicament liquide, e.g. insuline, peut être extraite du réservoir, i.e. de la poche interne, par un conduit de sortie alimenté par un orifice de sortie du réservoir.

La pompe de perfusion 10 est configurée pour délivrer le médicament, i.e. l'insuline, à dose contrôlée, par exemple des doses, tels des bolus ou autres, de quelques µL à une dizaines de µL.

Pour ce faire, la pompe de perfusion 10 est équipée d'un système de dosage 11.1 agencé en sortie de réservoir 11, de préférence associé au réservoir 11, afin de fournir les quantités d'insuline désirées. Le système de dosage 11.1 peut être motorisé et commandé par une électronique de commande (non montrée), par exemple une carte électronique à microprocesseur ou analogue. Le système de dosage 11.1 peut être alimenté par le conduit de sortie du réservoir 11.

Selon un mode réalisation, la pompe de perfusion 10 peut communiquer avec un appareil extérieur, tel un smartphone, afin de permettre à l'utilisateur de configurer les doses ou d'opérer d'autres choix, réglages, sélections, validations ou autres.

Afin de permettre la fourniture du fluide médicamenteux, i.e. de l'insuline, à un cathéter 20 ou analogue, la pompe de perfusion 10 est équipée d'un premier connecteur 13, aussi appelé connecteur femelle, auquel peut venir se coupler un second connecteur 23, appelé connecteur mâle, portée par l'extrémité amont 21.1, i.e. une extrémité libre, d'un tuyau flexible 21 faisant partie d'un cathéter 20, tel que détaillé ci-après.

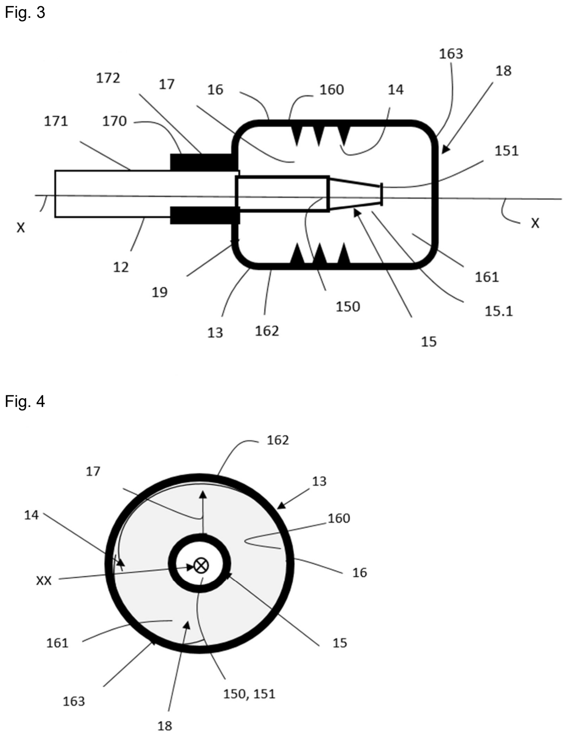

Dans le mode de réalisation de Fig. 1 et Fig. 3, le premier connecteur 13 ou connecteur femelle est agencé à une extrémité libre 12.1 d'un conduit flexible 12 intermédiaire, de préférence de courte longueur, i.e. de moins de 20 cm, de préférence moins de 10 cm, qui est relié fluidiquement au réservoir 11 de la pompe 10. Autrement dit, le conduit flexible 12 est agencé ici entre la pompe 10 et le premier connecteur 13. De préférence, le conduit flexible 12 est raccordé fluidiquement à une sortie du réservoir 11 de la pompe 10, préférentiellement à une sortie du système de dosage qui permet de prélever l'insuline dans le réservoir 11 de stockage de la pompe 10.

Utiliser un conduit flexible 12 intermédiaire peut faciliter, notamment pour certains patients, le couplage du cathéter 20 à la pompe 10, en particulier des connecteurs mâle et femelle l'un à l'autre, comme détaillé ci-après.

Toutefois, selon un autre mode de réalisation (non montré), le premier connecteur 13 ou connecteur femelle peut être agencé directement sur la pompe 10, c'est-à-dire sans utilisation d'un conduit flexible 12 intermédiaire. Le premier connecteur 13 est alors alimenté en insuline ou autre par le système de dosage fournissant les doses d'insuline désirées.

Comme illustré sur Fig. 3 et Fig. 4, le premier connecteur 13, i.e. le connecteur femelle, comprend un corps tubulaire 16, i.e. un corps principal externe, de préférence de forme générale cylindrique, d'axe longitudinal (XX).

Le premier connecteur 13 est typiquement en polymère, de préférence en polymère transparent.

Le corps tubulaire 16 est creux et sa paroi périphérique interne 160 délimite une chambre ou un volume interne 161, préférentiellement cylindrique, comprenant une extrémité avant ouverte 18 avec une ouverture large 18.1, et, à l'opposé, une extrémité arrière fermée par une paroi 19. Autrement dit, il a une forme générale de révolution, par exemple une forme de coupelle ou analogue.

L'extrémité avant ouverte 18 est bordée, i.e. entourée, par une bordure annulaire 163 du corps tubulaire 16. La bordure annulaire 163 forme une surface annulaire située à l'extrémité terminale avant du corps tubulaire 16, délimitant l'ouverture large 18.1 de l'extrémité avant ouverte 18.

Le corps tubulaire 16 est agencé coaxialement (axe XX) autour d'une tubulure interne 15, agencée dans la chambre interne 161, en étant séparés l'un de l'autre par un espacement annulaire 17 faisant partie de la chambre interne 161.

La tubulure interne 15 est donc contenue dans la chambre interne 161 mais a une longueur (selon axe XX) égale ou préférentiellement inférieure à celle de la chambre interne 161.

La tubulure interne 15 comprend un passage interne 150, i.e. un lumen, débouchant au niveau d'une sortie de fluide 151 permettant de délivrer le fluide, i.e. insuline. La sortie de fluide 151 de la tubulure interne 15 fait face à l'extrémité avant ouverte 18 de la chambre interne 161 du corps tubulaire 16 mais est située en retrait par rapport à cette dernière, c'est-à-dire que la sortie de fluide 151 de la tubulure interne 15 est positionnée dans la chambre interne 161. Le fluide provenant de la pompe 10 circule dans le passage interne 150 en direction de la sortie de fluide 151.

Selon un mode de réalisation, l'extrémité libre de tubulure interne 15 est au moins partiellement tronconique, c'est-à-dire que la tubulure interne 15 comprend une portion terminale 15.1 de forme tronconique comprenant la sortie de fluide 151. Cette portion tronconique permet d'améliorer l'étanchéité fluidique lorsqu'elle est insérée dans le passage interne 232, 232.1 du connecteur mâle 23 comme expliqué ci-après.

Selon un autre mode de réalisation, l'extrémité libre de la tubulure interne 15 est sensiblement cylindrique.

Par ailleurs, la tubulure interne 15 est en communication fluidique et alimenté en insuline par le conduit flexible 12 qui achemine l'insuline provenant du réservoir 11 de la pompe 10, via le système de dosage 11.1 associé au réservoir 11. Ici, le conduit flexible 12 vient se raccorder à la partie arrière du premier connecteur 13, à savoir à une portion arrière tubulaire 170 qui est solidaire du corps tubulaire 16 du premier connecteur 13, typiquement solidaire de la paroi arrière 19. La portion arrière tubulaire 170 forme en outre un renfort de fixation du conduit flexible 12. Le conduit flexible 12 peut aussi être texturé pour permettre une meilleure prise en main par l'utilisateur, c'est-à-dire que la surface externe du conduit peut présenter un relief antidérapant ou analogue.

La portion arrière tubulaire 170 présente un diamètre extérieur inférieur à celui du corps tubulaire 16 du premier connecteur 13. Elle est traversée par un canal interne 171 agencé coaxialement (XX) au passage interne 150 de la tubulure interne 15 et en communication fluidique l'un avec l'autre, via un orifice 172 traversant la paroi arrière 19 du corps tubulaire 16, de sorte que l'insuline convoyé par le conduit flexible 12 traverse successivement le canal interne 171 de la portion arrière tubulaire 170, l'orifice 172, et le passage interne 150 de la tubulure interne 15, avant de ressortir par l'orifice de sortie 151.

La portion tubulaire arrière 170, le corps tubulaire 16 et préférentiellement la tubulure interne 15 sont avantageusement formés d'une pièce unique, par exemple obtenue par injection-moulage ou toute autre technique adaptée, de matériau polymère, i.e. d'une matière plastique.

Par ailleurs, le corps tubulaire 16 du premier connecteur 13 comprend aussi un taraudage 14 aménagé dans la paroi périphérique interne 160 du corps tubulaire 16, c'est-à-dire du côté de la chambre interne 161. Il permet d'assurer un couplage par vissage des premier et second connecteurs 13, 23 l'un avec l'autre comme détaillé ci-après.

Par ailleurs, afin de garantir une meilleure manipulation du premier connecteur 13 par un utilisateur, en limitant le glissement de ses doigts lorsqu'il saisit et manipule le connecteur femelle 13, au moins une partie de la surface périphérique externe 161 du premier connecteur 13 est texturée, c'est-à-dire qu'elle comprend une ou des zones en relief, par exemple des striures ou analogue.

La délivrance au patient du fluide médicamenteux, i.e. de l'insuline, provenant de la pompe de perfusion 10 se fait via un cathéter 20 comprenant un tuyau flexible 21, typiquement en polymère transparent, qui permet de recueillir le fluide fourni par la pompe 10 et de le convoyer jusqu'à un hub ou plateforme de perfusion 30 comprenant une aiguille ou canule d'injection 31 ou analogue, permettant d'injecter le médicament à travers la peau P du patient, par exemple en sous-cutané, comme schématisé en Fig. 1.

De préférence, des moyens adhésifs 32, telle une surface collante, sont prévus sur une face inférieure, i.e. de dessous, du hub de perfusion 30 afin de permettre de coller le hub de perfusion 30 directement sur la peau P du patient et le maintenir en place pendant la perfusion.

Le raccordement mécanique et fluidique de l'extrémité aval 21.2 du tuyau flexible 21 du cathéter 20 au hub ou plateforme de perfusion 30 se fait préférentiellement via un système de couplage 22 spécifique, par exemple un connecteur clipsable.

Le cathéter 20 comprend donc le tuyau flexible 21, de préférence un conduit souple transparent, équipé du connecteur mâle 23 à son extrémité amont 21.1, et du système de couplage 22 au hub 30 à son extrémité aval 21.2.

En général, un cathéter 20 peut avoir une longueur allant jusqu'à environ 1 m.

Le tuyau flexible 21 du cathéter 20 est équipé du second connecteur 23, i.e. le connecteur mâle, qui est complémentaire du premier connecteur 13, i.e. le connecteur femelle, de sorte de pouvoir les coupler mécaniquement et fluidiquement l'un à l'autre en assurant une étanchéité de raccordement et un bon cheminement de l'insuline vers le hub de perfusion 30.

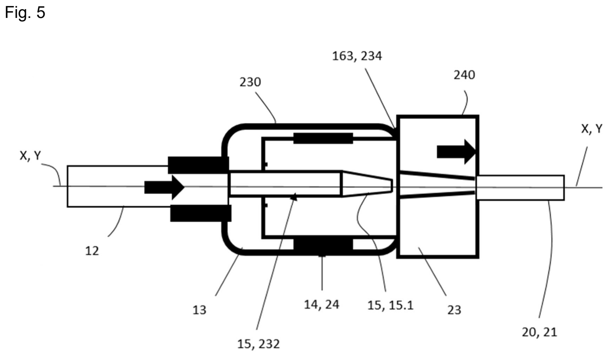

Plus précisément, le second connecteur 23 comprend un corps principal 23.1 comprenant une portion avant tubulaire 230 portant un filetage 24 sur sa surface périphérique externe 231, et une portion arrière 240 à laquelle vient se raccorder fluidiquement le tuyau flexible 21 du cathéter 20, comme illustré en Fig. 5. Le second connecteur 23 est typiquement en polymère, de préférence en polymère transparent, par exemple en MABS.

La portion avant tubulaire 230 et la portion arrière 240 sont traversées par un passage de fluide interne 232 (i.e. un lumen) s'étendant selon l'axe longitudinal (YY), permettant de relier fluidiquement une entrée de fluide 233 agencée sur la portion avant tubulaire 230, à une sortie de fluide portée par la portion arrière 240, de sorte d'acheminer le fluide, i.e. insuline, et le fournir au tuyau flexible 21 du cathéter 20.

L'entrée de fluide 233 est suffisamment large pour permettre d'y insérer la tubulure interne 15, en particulier la portion terminale 15.1 de forme tronconique, du premier connecteur 13.

De préférence, la portion avant 230 tubulaire est cylindrique.

Selon un mode de réalisation, le passage de fluide interne 232 du second connecteur 23 comprenant, lui aussi, une portion tronconique interne 232.1, c'est-à-dire qu'une partie de la paroi périphérique du passage de fluide interne 232 est aussi tronconique. Ainsi, l'étanchéité de connexion est améliorée étant donné que la portion terminale 15.1 de forme tronconique du premier connecteur 13 vient coopérer avec la paroi périphérique interne, également de forme tronconique, du passage de fluide interne 232 du second connecteur 23, ces deux portions étant de forme tronconique complémentaire.

La portion tronconique interne 232.1 peut par exemple être au moins partiellement agencée dans la portion arrière 240 du second connecteur 23 comme illustré en Fig. 2.

Selon un autre mode de réalisation, le passage de fluide interne 232 du second connecteur 23 a une forme sensiblement cylindrique et complémentaire de celle de l'extrémité libre de la tubulure interne 15 qui aussi sensiblement cylindrique de manière à, là encore, assurer un bon raccordement étanche de ces composants.

Par ailleurs, la portion arrière 240 du second connecteur 23 est avantageusement conformée pour être saisie à 2 doigts par l'utilisateur, en particulier être tenue entre pouce et index, en particulier elle comprend des zones aplaties à surface plane ou courbée, par exemple deux zones aplaties situées de part et d'autre de la portion arrière 240.

De préférence, la portion arrière 240 a une largeur ou un diamètre supérieur au diamètre de la portion avant 230 tubulaire, comme visible sur Fig. 5.

Le second connecteur 23 comprend, au niveau de la portion avant 230 tubulaire, un filetage 24 complémentaire du taraudage 14 du premier connecteur 13 de la pompe 10 de manière à pouvoir coupler par vissage les premier et second connecteurs 13, 23, l'un à l'autre, grâce à une coopération du filetage 24 et du taraudage 14.

Par ailleurs, le second connecteur 20 comprend aussi un épaulement 234 pouvant former butée, de préférence un épaulement annulaire 234 comme illustré en Fig. 2. Cet épaulement annulaire 234 est porté par la portion arrière tubulaire 240. Il constitue une surface avant annulaire de la portion arrière 240 du second connecteur 20.

Selon un mode de réalisation, l'épaulement annulaire 234 est destiné à venir se positionner en regard de la bordure annulaire 162 du premier connecteur 10 et préférentiellement prendre appui, i.e. venir en butée, contre la bordure annulaire 162 du premier connecteur 10 délimitant l'extrémité avant ouverte 18 du premier connecteur 10, lorsque les deux connecteurs 13, 23 sont couplés l'un à l'autre, comme montré en Fig. 5. Dans ce cas, lorsque l'épaulement annulaire 234 du second connecteur 20 vient en butée contre la bordure annulaire 162 du premier connecteur 13, la rotation relative des connecteurs 13, 23 l'un relativement à l'autre peut être stoppée, afin de limiter la rotation entre 60° et 180°, comme expliqué ci-après.

Il est à noter que la portion arrière tubulaire 240 du second connecteur 23 peut aussi comprendre, comme la portion arrière tubulaire 170 du premier connecteur 13, un renfort de fixation du tuyau souple 21 du cathéter 20.

Comme déjà dit, lorsque les deux connecteurs 13, 23 sont couplés l'un à l'autre, comme montré en Fig. 5, la tubulure interne 15 du premier connecteur 13 vient s'insérer, de manière étanche, dans le passage de fluide interne 232 du second connecteur 23 de manière à assurer une continuité et une étanchéité fluidiques entre le passage interne 150 du premier connecteur 13 et le passage de fluide interne 232 du second connecteur 23 et permettre ainsi une circulation du fluide, i.e. insuline, en direction du tuyau flexible 21 du cathéter 20, et ensuite vers le hub 30.

A cette fin, la région d'extrémité 15.1 de la tubulure interne 15 du premier connecteur 13 est conformée extérieurement pour faciliter son insertion dans le passage de fluide interne 232 du second connecteur 23 et/ou améliorer l'étanchéité gazeuse avec la paroi périphérique interne 232.1 du passage de fluide interne 232 du second connecteur 23, lorsqu'elle est insérée dans ledit passage de fluide interne 232 du second connecteur 23, en particulier ils comprennent des portions ayant des formes complémentaires, par exemple des formes tronconiques ou autres, en particulier la région d'extrémité 15.1 de la tubulure interne 15 du premier connecteur 13 a une forme externe complémentaire de celle de la portion interne 232.1 du passage de fluide interne 232 du second connecteur 23 de manière à assurer un raccordement étanche entre ces éléments et de préférence leur verrouillage ou blocage l'un dans l'autre

Lorsque les connecteurs 13, 23 sont insérés l'un dans l'autre, leur fixation et leur verrouillage se fait par vissage, c'est-à-dire via un déplacement en rotation (R) relatif de l'un par rapport à l'autre, selon un angle de rotation que lui applique l'utilisateur.

Selon la présente invention, l'angle de rotation est limité, à savoir est compris entre 100° et 200°, afin de faciliter le raccordement du cathéter 20 à la pompe de perfusion 10 et par ailleurs assurer un raccordement efficace et une bonne étanchéité fluidique entre les connecteurs 13, 23.

Avantageusement, l'angle de rotation permettant un vissage efficace
est inférieur ou égal à 195° environ, de préférence inférieur ou égal à 190° environ, typiquement inférieur ou égal à 185° environ, voire inférieur ou égal à 180° environ, et/ou d'au moins 105° environ, de préférence d'au moins 110° environ, de préférence encore d'au moins 120° environ, de préférence encore 130° environ, de préférence encore d'au moins 135° environ, voire d'au moins 140° environ, par exemple entre 145° et 195° environ, ou entre 150° et 190° environ.

De manière préférée, l'angle de rotation est compris entre 150° et 190° environ, de préférence encore compris entre 155° et 180° environ.

Lorsque les connecteurs 13, 23 sont mis en rotation relatif l'un par rapport à l'autre, le filetage 24 du second connecteur 23 et le taraudage 14 du premier connecteur 13 viennent coopérer l'un avec l'autre pour assurer le vissage.

Selon un mode de réalisation, les connecteurs 13, 23 peuvent avoir chacun une longueur d'environ 1,5 à 2,5 cm.

Selon un mode de réalisation, la rotation appliquée peut se poursuivre jusqu'à ce que l'épaulement annulaire 234 du second connecteur 23 vienne en butée contre la bordure annulaire 162 du premier connecteur 13. Il n'est plus alors possible de continuer la rotation, ce qui limite la course en rotation à moins de 200°, de préférence à moins de 190°, par exemple à de l'ordre d'environ 180° à 185°, comme susmentionné.

Selon un autre mode de réalisation, la rotation appliquée de moins de 200° peut se poursuivre jusqu'à ce que les tronçons complémentaires, par exemple tronconiques 15.1, 232.1, des connecteurs 13, 23 viennent s'insérer de manière étanche l'un dans l'autre.

Selon encore un autre mode de réalisation, les filetage 24 et taraudage 14 des connecteurs 13, 23 peuvent être configurés et/ou dimensions et/ou conçus pour que la rotation ne puisse excéder 200° ou un angle inférieur donné.

En d'autres termes, la course en rotation peut s'arrêter, selon le mode de réalisation choisi, par exemple :
- lorsque l'épaulement annulaire 234 du second connecteur 23 vient prendre appui, i.e. en butée, contre la bordure annulaire 162 délimitant l'extrémité avant ouverte 18 du premier connecteur 10, et/ou
- lorsque les portions complémentaires, par exemple tronconiques 15.1, 232.1 de la tubulure interne 15 du premier connecteur 13 et du passage de fluide interne 232 du second connecteur 23 sont associées l'une à l'autre, i.e. insérées de manière étanche l'une dans l'autre, et/ou
- lorsque le filetage 24 et le taraudage 14 sont vissés au maximum l'un dans l'autre, par exemple jusqu'à obtenir un verrouillage ou blocage « à force ».

Bien entendu, d'autres moyens peuvent être prévus pour stopper la rotation comme une ou des butées, des épaulements ou similaires.

D'une façon générale, un utilisateur peut facilement opérer le couplage et vissage en tenant par exemple le premier connecteur 13 dans l'une de ses mains, par exemple dans sa main gauche pour une personne droitière, et maintenir le second connecteur 23 dans son autre main, par exemple sa main droite dans ce cas, et opérer, après début d'insertion des deux connecteurs l'un dans l'autre par rapprochement l'un de l'autre, un vissage des connecteurs 13, 23 l'un avec l'autre, selon une course en rotation entre 100° et 200°, de préférence d'au moins 110°, voire plus, et de moins de 195° ou moins, par exemple de l'ordre de 170° à 190°, comme expliqué ci-avant, par exemple en saisissant et maintenant la portion arrière 240 du second connecteur 23 entre 2 doigts, tels qu'entre pouce et index, et en lui appliquant un mouvement de rotation dans le sens horaire, i.e. sens de vissage.

Dès que le vissage n'est plus possible du fait par exemple de l'épaulement annulaire 234 du second connecteur 20 qui vient en butée contre la bordure annulaire 162 du premier connecteur 13 ou des portions tronconiques 15.1, 232.1 des connecteurs 13, 23 coopérant l'une avec l'autre, l'utilisateur cesse d'appliquer la rotation au second connecteur 23 et peut être certain que le couplage s'est opéré de manière efficace.

A l'inverse, le découple des connecteurs 13, 23, notamment pour remplacer un cathéter 20 usagé par un cathéter neuf, se fait en procédant à l'inverse, c'est-à-dire en appliquant un mouvement de dévissage dans le sens antihoraire jusqu'à obtenir une dissociation du filetage 24 et du taraudage 14, et une libération des deux connecteurs 13, 23.

Selon l'invention, le fait que le couplage par vissage des premier et second connecteurs 13, 23, l'un avec l'autre, comprenne un déplacement en rotation (R) relatif de l'un par rapport à l'autre, selon un angle de rotation d'au moins 100° et inférieur à 200°, permet d'assurer un raccordement efficace et une bonne étanchéité fluidique entre les premier et second connecteurs 13, 23, y compris lorsque leur raccordement est opéré par des patients présentant des complications liées à leur diabète, notamment des troubles ou complications visuelles ou micro ou macro vasculaires réduisant leur dextérité par exemple.

## Revendications

1. Ensemble (1) d'administration d'un médicament liquide, de préférence de l'insuline, comprenant :
- une pompe (10) de perfusion comprenant un réservoir (11) pour stocker le médicament liquide, et un premier connecteur (13) comprenant un taraudage (14), en communication fluidique avec le réservoir (11), et
- un cathéter (20) comprenant un tuyau flexible (21) équipé d'un second connecteur (23) comprenant un filetage (24) complémentaire du taraudage (14) du premier connecteur (13) de manière à pouvoir coupler par vissage les premier et second connecteurs (13, 23), l'un à l'autre, et ainsi raccorder fluidiquement le cathéter (20) à la pompe (10),
et dans lequel :
- le premier connecteur (13) équipant la pompe (10) comprend un corps tubulaire (16) agencé coaxialement (XX) autour d'une tubulure interne (15) en étant espacé (17) l'un de l'autre en définissant une chambre interne (161), ladite tubulure interne (15) comprenant un passage interne (150) débouchant au niveau d'une sortie de fluide (151) faisant face à l'extrémité avant ouverte (18) de la chambre interne (161) du corps tubulaire (16), et
- le second connecteur (23) équipant le cathéter (20) comprend une portion avant tubulaire (230) et un passage de fluide interne (232) reliant fluidiquement une entrée de fluide (233) agencée sur la portion avant tubulaire (230), au tuyau flexible (21) du cathéter (20),
**caractérisé en ce que** :
- le taraudage (14) est agencé dans la paroi interne (160) du corps tubulaire (16) du premier connecteur (13) équipant la pompe (10),
- le filetage (24) est porté par la surface périphérique externe (231) de la portion avant tubulaire (230) du second connecteur (23) équipant le cathéter (20), et
- lorsque les premier et second connecteurs (13, 23) sont couplés l'un à l'autre :
▪ le filetage (24) du second connecteur (23) et le taraudage (14) du premier connecteur (13) coopèrent l'un avec l'autre, pour assurer le couplage par vissage des premier et second connecteurs (13, 23), l'un à l'autre, avec déplacement en rotation (R) relatif de l'un par rapport à l'autre, selon un angle de rotation compris entre 100° et 200°, et
▪ la tubulure interne (15) du premier connecteur (13) vient se loger de manière étanche, dans la portion avant tubulaire (230) du second connecteur (23).

2. Ensemble selon la revendication 1, **caractérisé en ce que** l'angle de rotation est inférieur ou égal à 195°, de préférence inférieur ou égal à 190°, de préférence encore inférieur ou égal à 185°.

3. Ensemble selon la revendication 1, **caractérisé en ce que** l'angle de rotation est d'au moins 110°, de préférence d'au moins 120°.

4. Ensemble selon l'une des revendications 1 à 3, **caractérisé en ce que** l'angle de rotation est compris entre 130° et 180°.

5. Ensemble selon la revendication 1, **caractérisé en ce que** le premier connecteur (13) de la pompe (10) de perfusion est agencé à une extrémité libre (12.1) d'un conduit flexible (12) relié fluidiquement au réservoir (11).

6. Ensemble selon la revendication 1, **caractérisé en ce que** le second connecteur (23) comprend en outre une portion arrière (240) conformée pour être saisie à 2 doigts par l'utilisateur, en particulier être tenue entre pouce et index.

7. Ensemble selon la revendication 6, **caractérisé en ce que** la portion arrière (240) du second connecteur (23) a une forme aplatie.

8. Ensemble selon la revendication 1, **caractérisé en ce que** le second connecteur (23) comprend un épaulement annulaire (234) venant se positionner en regard d'une bordure annulaire (163) du premier connecteur (13), lorsque les premier et second connecteurs (13, 23) sont couplés l'un à l'autre.

9. Ensemble selon la revendication 8, **caractérisé en ce que** l'extrémité avant ouverte (18) du premier connecteur (13) est délimitée par la bordure annulaire (163).

10. Ensemble selon la revendication 1, **caractérisé en ce que** la tubulure interne (15) du premier connecteur (13) comprend une portion terminale (15.1) de forme donnée comprenant la sortie de fluide (151), et le passage de fluide interne (232) du second connecteur (23) comprend une portion interne (232.1) complémentaire de la portion terminale (15.1) de forme donnée de la tubulure interne (15) du premier connecteur (13), de préférence la portion terminale (15.1) est de forme tronconique ou cylindrique.

11. Ensemble selon la revendication 1, **caractérisé en ce que** le réservoir (11) de la pompe (10) est amovible et/ou comprend un boitier externe rigide contenant une poche interne souple destinée à recevoir le médicament liquide, de préférence de l'insuline.

12. Ensemble selon la revendication 1, **caractérisé en ce que** le corps tubulaire (16) du premier connecteur (13) équipant la pompe (10) comprend une paroi externe texturée.

13. Cathéter (20) d'un ensemble (1) d'administration de médicament liquide, de préférence de l'insuline, selon l'une des revendications précédentes comprenant un tuyau flexible (21) équipé du second connecteur (23) et d'un système de couplage (22) à une plateforme de perfusion (30) comprenant une aiguille de perfusion (31) servant à délivrer le médicament liquide.

14. Ensemble de perfusion de médicament liquide (10, 20, 30) comprenant :
- la pompe (10) de perfusion et le cathéter (20) d'un ensemble (1) d'administration de médicament liquide selon l'une des revendications 1 à 12, et
- une plateforme de perfusion (30) comprenant une aiguille de perfusion (31) servant à délivrer le médicament liquide,
dans lequel ladite aiguille de perfusion (31) est en communication fluidique avec le réservoir de la pompe (10) de perfusion, via le cathéter (20).
